# EUROPÄISCHE PATENTANMELDUNG

(11) **EP 1 391 183 A1**
(43) Veröffentlichungstag der Anmeldung: **25.02.2004**
(21) Anmeldenummer: 03018672.0
(22) Anmeldetag: 21.08.2003
(51) Int. Cl.: A61C 13/267, A61K 6/083

(54) **Dentalprothese mit metallfreien Verankerungselementen**

(30) Priorität: 23.08.2002 DE 10238833
(71) Anmelder: Ivoclar Vivadent AG, 9494 Schaan (LI)
(72) Erfinder: Zappini, Gianluca Dr., 38069 Torbole sul Garda (TN) (IT); Zanghellini, Gerhard, 9494 Schaan (LI); Rheinberger, Volker Dr., 9490 Vaduz (LI)
(74) Vertreter: UEXKÜLL & STOLBERG

(57) **Zusammenfassung**

Die Erfindung betrifft Dentalprothesen, die mindestens ein metallfreies Verankerungselement aus Kunststoffmaterial mit einem Biege-E-Modul von mindestens 10 GPa und einer maximalen Dehnung von mindestens 0,8 % aufweisen.

## Beschreibung

Die Erfindung betrifft Dentalprothesen, die metallfreie Verankerungselemente aufweisen.

Aus ästhetischen Gründen werden metallhaltige Dentalprothesen zunehmend durch metallfreie Prothesen ersetzt. Als Materialien zur Herstellung metallfreier Prothesen haben sich Kompositwerkstoffe bewährt, die partikel- oder faserförmige Füllstoffe enthalten.

Die EP 0 230 394 A2 offenbart orthodontische Apparaturen, wie z.B. Zahnklammern, aus faserverstärktem Kunststoff, der vorzugsweise die Form von Drähten hat.

Die EP 0 389 552 B1 offenbart temporäre und permanente Brücken auf der Basis faserverstärkter Kunststoffe. Zur Herstellung der Brücken werden Kunststoffzähne an einem Streifen aus glasfaserverstärktem Kunststoff befestigt und dieser dann durch Ankleben an die der zu schließenden Lücke benachbarten Zähne im Mundes des Patienten angebracht.

Die US 5,098,304 beschreibt die Herstellung von Dentalrestaurationen auf der Basis von glasfaserverstärkten Kompositmaterialien.

Aus der US 5,839,900 ist ein zweistufiges Verfahren zur Herstellung dentaler Prothesen bekannt, das die Herstellung eines faserverstärkten Gerüsts und die anschließende Verblendung des Gerüsts mit einem Kompositmaterial umfaßt (vgl. auch K. H. Körber et al., DSL **3/96** 12).

Die US 5,062,799 offenbart dentale Verblendmaterialien, die aus beidseitig mit Kompositmaterial beschichtetem Glasfasermaterial bestehen und auf die Zahnoberfläche aufgeklebt werden.

Die US 5,176,951 betrifft dentale Restaurationen, die mit gewebten Stoffen auf der Basis von Aramid- oder Polyethylenfasern verstärkt sind.

Die DE 38 21 091 A1 beschreibt die Herstellung dentaler Prothesen mit metallischen Gußklammern bei dem eine lichtpolymerisierbare Harzzubereitung, die eine ethylenisch ungesättigte Verbindung und organischen Füllstoff enthält, an einem Modell zu einer Klammer geformt wird, die nach der Härtung zur Herstellung einer Gußform zur Anfertigung der metallischen Gußklammern nach dem Wachsausschmelzverfahren dient.

Von der Firma Micro Dental Laboratories werden unter der Bezeichnung Dental D® monomerfreie Werkstoffe auf der Basis von Acetal-Harz vertrieben, die sich zur Herstellung metallfreier Dentalprothesen eignen und die Herstellung von Partialprothesen mit zahnfarbenen Verankerungselementen ermöglichen sollen.

Die Firma Trident Dental Laboratories vertreibt für den gleichen Zweck unter der Bezeichnung Thermoflex® ein Material, das eine monomerfreie, kristalline Struktur aufweisen soll und ebenfalls auf Acetal-Harz basiert.

Bei vielen dentalen Anwendungen konnten mit Kunststoffen und Kompositen gute Ergebnisse erzielt wurden. Beispielsweise zeichnen sich Kronen und Brücken auf der Basis von faserverstärkten Kompositen, die mit glaspulverhaltigen Kunststoffen verblendet sind, durch eine hohe Belastbarkeit aus, so daß ohne Nachteile auf Metallbewehrungen verzichtet werden kann. In anderen Bereichen konnten jedoch mit Kunststoffen bisher nicht die Eigenschaften von Metallen erreicht werden. So sind für die Befestigung von Teilprothesen am Restgebiß des Patienten Metallklammern immer noch das Mittel der Wahl, wenn eine sichere Verankerung im Vordergrund steht. Durch die Verwendung von Klammern auf der Basis von Acetal-Harzen kann nicht die Sicherheit von Metallklammern erreicht werden. Andererseits sind Metallklammern häufig relativ unflexibel, was das Einsetzen und Herausnehmen der Prothese erschwert und das Risiko einer Beschädigung der Verankerungszähne birgt. Zudem führt das mit dem Einsetzen und Herausnehmen der Restauration verbundene Verformen der Klammer auf Grund der relativ geringen Elastizität von Metallklammern leicht zu plastischen Verformungen der Klammer und Materialermüdungen.

Der Erfindung liegt demnach die Aufgabe zu Grunde, Dentalprothesen zur Verfügung zu stellen, die ohne Beschädigung von Zähnen und Prothese leicht eingesetzt und wieder entfernt und die ohne die Verwendung von Metallen sicher im Munde des Patienten verankert werden können.

Diese Aufgabe wird durch Dentalprothesen gelöst, die mindestens ein Verankerungselement aus Kunststoffmaterial aufweisen, das einen Biege-E-Modul von mindestens 10 GPa und eine maximale Dehnung vor Eintreten einer plastischen Verformung von mindestens 0,8 % aufweist. Bevorzugt sind Verankerungselemente aus Kunststoffmaterial mit einem Biege-E-Modul von 10 bis 80 GPa und einer maximalen Dehnung von 0,8 bis 4 %. Da Verankerungselemente für dentale Prothesen oft ein klammerförmiges Aussehen haben, werden sie im Folgenden auch als Klammern bezeichnet.

Erfindungsgemäß werden unter Dentalprothesen Voll- und vorzugsweise Teilprothesen verstanden. Von Vollprothesen spricht man bei herausnehmbarem Zahnersatz, der der Wiederherstellung des Kauorgans bei völliger Zahnlosigkeit eines Kiefers dient. Vollprothesen können mit Verankerungselementen an Implantaten befestigt werden. Bei Teilprothesen oder partiellen Prothesen handelt es sich um herausnehmbaren Zahnersatz zur Wiederherstellung des Kauorgans bei teilweisem Zahnverlust, der am Restgebiß des Patienten und/oder entsprechenden Implantaten befestigt werden kann.

Partialprothesen lassen sich in verschiedene Bauelemente zerlegen. Den eigentlichen Zahnersatz stellen der oder die Sättel dar. Als solche bezeichnet man die mit künstlichen Zähnen versehenen Ersatzstücke, die im Bereich einer unterbrochenen oder verkürzten Zahnreihe den zahnlosen Kieferabschnitten aufliegen. Enthält eine Teilprothese mehrere Sättel, so werden diese durch Verbindungselemente miteinander verbunden. Die Sättel und ihre Verbindungselemente ergeben zusammen die Prothesenbasis. Bei Prothesen mit nur einem Sattel wird dieser als Basis bezeichnet. Durch Adhäsion allein hat die Basis einer Teilprothese im Munde keinen ausreichenden Halt. Sie wird daher in der Regel mit Verankerungselementen am Restgebiß oder Implantaten befestigt. Die erfindungsgemäßen Dentalprothesen weisen eine Prothesenbasis mit mindestens einem, gewöhnlich 1 bis 3 Sätteln und mindestens ein Verankerungselement, gewöhnlich 2 bis 8 Verankerungselemente auf. Da Prothesen immer Einzelstücke sind, die auf die jeweilige Patientensituation angepaßt werden müssen, lassen sich keine Angaben zur bevorzugten Anzahl an Sätteln und Verankerungselementen machen.

Die Erfindung basiert auf der überraschenden Erkenntnis, daß für eine sichere Verankerung von Restaurationen im Mund des Patienten weniger die Bruchfestigkeit des Materials der Klammer als vielmehr eine ausgewogene Kombination von Biege-E-Modul und maximaler Dehnung verantwortlich ist. Diese Eigenschafstkombination läßt sich auf besonders vorteilhafte Weise durch Klammern aus Kunststoffmaterial verwirklichen, das ein polymeres Matrixmaterial enthält, in das ein faserförmiger Füllstoff eingebettet ist (faserverstärkter Kunststoff). Das faserverstärkte Kunststoff ist vorzugsweise mit einem Polymermaterial verblendet. Der faserverstärkte Kunststoff wird daher im Folgenden auch als Kernmaterial bezeichnet. Das Verblendmaterial enthält keinen faserförmigen Füllstoff.

Das Kunststoffmaterial, d.h. das Kernmaterial bzw. die Kombination aus Kern- und Verblendmaterial, weist einen Biege-E-Modul von mindestens 10 GPa und vorzugsweise von 10 bis 80 GPa, besonders bevorzugt 10 bis 50 GPa, und eine maximale Dehnung von mindestens 0,8 %, vorzugsweise von 0,8 bis 4 %, besonders bevorzugt 2,0 bis 3,5 % auf. Der Biege-E-Modul des Klammermaterials wird analog zu DIN/ISO 178 ermittelt. Unter der maximalen Dehnung wird die Dehnung verstanden, die das Material ohne plastische Verformung und mechanische Schädigung (z.B. Bruch einer Schicht oder deren Abplatzen) überstehen kann. Auch diese wird gemäß DIN/ISO 178 bestimmt.

Biege-E-Modul und maximale Dehnung werden vorzugsweise so gewählt, daß die Flexibilität des Materials, die als der Quotient aus maximaler Dehnung in % und Biege-E-Modul in GPa definiert ist, im Bereich von 0,4 ·10⁻³ bis 15 · 10⁻³ GPa⁻¹, vorzugsweise 1 · 10⁻³ bis 5 · 10⁻³ GPa⁻¹ liegt.

Biege-E-Modul und maximale Dehnung sind Stoffkonstanten, die für einen gegebenen Werkstoff charakteristisch sind. Im Fall von Materialkombinationen, wie Kombinationen aus Kernmaterial und Verblendmaterial, lassen sich für das Kern- und das Verblendmaterial unterschiedliche Werte für den Biege-E-Modul und die maximale Dehnung angeben. Die Werte des Klammermaterials werden durch die Werte der zur Herstellung der Klammer verwendeten Materialien und durch deren Schichtdicken bestimmt. Beispielsweis läßt sich ein Kunststoffmaterial mit einem wie oben definierten Biege-E-Modul dadurch erhalten, daß eine relativ dicke Schicht eines Kernmaterials mit relativ niedrigem Biege-E-Modul mit einer relativ dünnen Schicht Verblendmaterial kombiniert wird, oder aber, indem eine relativ dünne Schicht eines Kernmaterials mit relativ hohem Biege-E-Modul mit einer relativ dicken Schicht an Verblendmaterial kombiniert wird, wobei unterstellt wird, daß das Verblendmaterial, das keinen faserförmigen Füllstoff enthält, einen geringeren Biege-E-Modul aufweist als das Kernmaterial. Die Messung von Biege-E-Modul und maximaler Dehung erfolgt jeweils an dem Klammermaterial, d.h. im Fall von Materialkombinationen werden diese analog den angegebenen DIN/ISO-Normen gemessen. Bei Klammern, die ausschließlich aus dem Kernmaterial bestehen, sind die Klammerwerte mit den Werten des Kernmaterials identisch.

Erfindungsgemäß ist die Verwendung von Kombinationen von mindestens zwei Kunststoffen, d.h. von mindestens einem Kernmaterial und mindestens einem Verblendmaterial, zur Herstellung der Klammern bevorzugt, da durch die Verblendung ein Schutz der Fasern des Kernmaterials vor Beschädigungen beim Einsetzen und Herausnehmen der Prothese erzielt und damit eine Reizung des Zahnfleischs durch freie Faserenden verhindert wird. Zudem ist durch die Kombination von Kern- und Verblendmaterial eine gezielte Einstellung von Biege-E-Modul, Dehnung und Flexibilität möglich. Die Verwendung von Verblendmaterialien ohne anorganische Füllstoffe ist bevorzugt, da sich ohne anorganische Füllstoffe günstigere Werte für die Flexibilität erzielen lassen.

Zur Verblendung des Kernmaterials können ein oder mehrere, vorzugsweise 1 bis 3 Verblendmaterialien eingesetzt werden. Durch die Verwendung unterschiedlich gefärbter Verblendmaterialien läßt sich beispielsweise eine besonders gute farbliche Anpassung der Klammer an die benachbarten natürlichen Zähne erzielen.

Gemäß einer bevorzugten Ausführungsform werden Kernmaterialen mit einem Biege-E-Modul von mehr als 20 GPa, besonders bevorzugt 30 bis 100 GPa, und einer maximalen Dehnung von mehr als 1 %, besonders bevorzugt 1,5 bis 3,0 % verwendet. Die Verblendmaterialien weisen vorzugsweise ein Biege-E-Modul von 2 bis 15 GPa, besonders bevorzugt 2 bis 5 GPa, und eine maximale Dehnung von mehr als 1 %, besonders bevorzugt 2 bis 5 % auf.

Als Kernmaterialen werden faserverstärkte Kunststoffe verwendet, d.h. Kunststoffe, die eine organische, polymere Matrix, einen darin eingebetteten faserförmigen Füllstoff und ggf. partikulären Füllstoff enthalten. Der partikuläre Füllstoff kann organischer oder anorganischer Natur sein. Die Kernmaterialen werden durch die Polymerisation entsprechender ungehärteter Ausgangsmaterialien erhalten, die neben faserförmigem und ggf. partikulärem Füllstoff ein polymerisierbares Bindemittel und Initiatoren für die radikalische Polymerisation sowie ggf. weiter Hilfsstoffe und Additive enthalten. Diese Ausgangsmaterialien werden im folgenden auch als härtbare Kernmaterialien bezeichnet.

Die in der Erfindung eingesetzten Bindemittel enthalten mindestens ein polymerisierbares Monomer, Oligomer und/oder Makromonomer. Oligomere sind aus mindestens 5, gewöhnlich aus 50 bis 300 Monomerbausteinen aufgebaut.

Erfindungsgemäß geeignete Monomere werden z.B. in der DE 198 18 210 C2, Seite 4 beschrieben. Bevorzugte monofunktionelle Monomere sind Methyl(meth)acrylat, Isobutyl(meth)acrylat, Butoxymethyl(meth)acrylat, 2-Ethylhexyl(meth)acrylat, Isodecyl(meth)acrylat, Phenoxymethyl(meth)acrylat, Ethyl(meth)acrylat, Butyl (meth) acrylat, Benzyl (meth) acrylat, Phenyl(meth)acrylat und Tetrahydrofurfuryl(meth)acrylat, Cyclohexyl-(meth)acrylat, Hydroxyethyl(meth)acrylat (HEMA), Glycerinmono(meth)acrylat. Unter monofunktionellen Monomeren werden Monomere mit einer radikalisch polymerisierbaren Gruppe verstanden.

Gemäß einer bevorzugten Ausführungsform enthält das Bindemittel mindestens ein Monomer mit zwei oder mehr radikalisch polymerisierbaren Gruppen. Solche Verbindungen wirken bei der Polymerisation als Vernetzer. Bevorzugte Monomere mit zwei radikalisch polymerisierbaren Gruppen sind 1,4-Butandioldi(meth)acrylat, 1,6-Hexandioldi(meth)acrylat, 1,9-Nonandioldi(meth)acrylat, Decanmethylenglykoldimethacrylat, Bis-[4-(meth)acryloxy-2-hydroxypropyloxyphenyl]propan, Polyethylenglycol(meth)acrylate (PEG-(Meth)acrylate), beispielsweise auf Basis von PEG 300, 400 oder 1000, Bisphenol-A-di(meth)acrylat, insbesondere Ethylenglycoldi(meth)acrylat, Triethylenglycoldi-(meth)acrylat, Dipropylenglycoldi(meth)acrylat, Bis-GMA (2,2-Bis-4-(3-(meth)acryloxy-2-hydroxypropyl)-phenylpropan), 1,1,6-Trimethylhexamethylenurethandi (meth)acrylat, Urethandi (meth)acrylat (UDMA, Reaktionsprodukt von Hydroxyethyl(meth)-acrylat oder Hydroxypropyl(meth)acrylat mit 2,2,4-Trimethylhexyl-1,6-diisocyanat), Glycerindi- und -tri(meth)acrylat.

Bevorzugte Monomere mit mehr als zwei radikalisch polymerisierbaren Gruppen sind Trimethylolpropantri(meth)acrylat, Pentaerythrittetra(meth)acrylat und Dipentaerythritpenta(meth)acrylat.

Bei allen angegebenen Monomeren sind die entsprechenden Methacrylatderivate besonders bevorzugt. Ganz besonders bevorzugt sind Hydroxyethyl(meth)acrylat und Glycerindi(meth)acrylat (GDMA).

Als faserförmige Füllstoffe eignen sich besonders keramische Fasern, organische Fasern, wie Aramid- und Polyethylenfasern, Borfasern, Kohlenstoffasern und insbesondere Glasfasern. Die faserförmigen Füllstoffe werden vorzugsweise in Form von Langfasern eingesetzt. Kernmaterialien, die 43 bis 70 Gew.-% faserförmigen Füllstoff enthalten, sind bevorzugt. Die Fasern weisen vorzugsweise einen Durchmesser von 10 bis 30 µm auf. Unter Langfasern werden Fasern verstanden, deren Länge mindestens das 500-fache des Durchmessers, vorzugsweise das 1.000- bis 10.000-fache des Durchmessern beträgt.

Als partikuläre Füllstoffe sind besonders Quarz-, Glaskeramikoder Glaspulver, Aluminium- und Siliziumoxidpulver, Pulver auf der Basis von Silikatgläsern, wie Bariumsilikat-, Li/Al-Silikatgläsern und Bariumgläsern, sowei Mischungen daraus geeignet. Diese Füllstoffe werden als Pulver mit einer mittleren Partikelgröße von vorzugsweise 0,1 bis 50 µm, insbesondere 1 bis 20 µm eingesetzt.

Darüber hinaus sind im Rahmen der Erfindung auch die sogenannten Kompositfüller einsetzbar, die durch Polymerisation einer Mischung eines der oben genannten anorganische Füllstoffe und einem Bindemittel und anschließendes Mahlen des gehärteten Polymerisats erhalten werden können.

Zusätzlich kann das Kernmaterial bzw. dessen ungehärtetes Ausgangsmaterial Pigmente, vorzugsweise anorganische Farbpigmente auf Oxidbasis, Röntgenopazitätsmittel, vorzugsweise Ytterbiumfluorid, Thixotropierungsmittel, wie pyrogene und/oder gefällte Kieselsäuren, Beschleuniger, beispielsweise Metallsalze und Komplexverbindungen, wie Kupferacetat, Kupferacetylacetonat, Kupfersalicylat, Co-EDTA-Komplex, und weitere Zusatz- und Hilsstoffe enthalten. Kieselsäuren werden überlicherweise in einer Menge von bis zu 20 Gew.-%, vorzugsweise 1 bis 10 Gew.-% verwendet, bezogen auf die Masse des Kernmaterials.

Als Polymerisatonsinitiatoren eignen sich alle Initiatoren der radikalischen Polymerisation, wie Redox-Systeme und Initiatoren für die Lichthärtung.

Bevorzugte Redoxinitiatoren (oxidierende Stoffe) sind Kobalt(III)chlorid, Peroxide, wie tert.-Butylhydroperoxid, Eisen(III)chlorid, Perborsäure und ihre Salze, Permanganate und Persulfatanionen. Wasserstoffperoxid kann ebenfalls verwendet werden. Ganz besonders bevorzugte Initiatoren sind Benzoxylperoxid (BPO) und Lauroylperoxid. Bei gleichzeitiger Verwendung von Photoinitiatoren können Wechselwirkungen mit den oben genannten oxidierenden Stoffe auftreten, so daß Photoinitiator und Redoxinitiator aufeinander abgestimmt werden sollten. Die Redoxinitiatoren können allein zur Härtung der polymerisierbaren Massen eingesetzt werden oder vorzugsweise in Kombination mit Aktivatoren.

Bevorzugte Aktivatoren (reduzierende Stoffe) sind Amine, insbesondere Diethanol-p-Toluidin, Ascorbinsäure, Barbitursäurederivate, Kobalt(II)chlorid, Eisen(II)chlorid, Eisen(II)sulfat, Hydrazin, Oxalsäure, Thioharnstoff und Salze von Dithionit- oder Sulfitanionen. Ganz besonders bevorzugte Aktivatoren sind Diethanol-p-Toluidin, Ascorbinsäure und Benzylphenylbarbitursäure (BPBS).

Das am meisten bevorzugte Redox-System ist BPO/BPBS.

Bevorzugte Photoinitiatoren sind Benzoinether, Dialkylbenzilketale, Dialkoxyacetophenone, Acylphosphinoxide, α-Diketone, wie 9,10-Phenanthrenchinon, Diacetyl, Furyl, Anisil, 4,4'-Dichlorbenzil, 4,4'-Dialkoxybenzil und insbesondere Camperchinon. Diese Photoinitatoren können allein oder in Kombination eingesetzt werden. Auch Mischungen von Photo- und Redoxinitiatoren sind geeignet.

Unabhängig voneinander wählbare bevorzugte Mengenbereiche für die einzelnen Komponenten des Kernmaterials bzw. dessen ungehärteten Ausgangsmaterials sind:
25 bis 52 Gew.-%, vorzugsweise 25 bis 40 Gew.-%, besonders bevorzugt 30 bis 40 Gew.-% polymerisierbares Bindemittel,
43 bis 70 Gew.-%, vorzugsweise 50 bis 70 Gew.-%, besonders bevorzugt 55 bis 65 Gew.-% faserförmiger Füllstoff,
3 bis 8 Gew.-%, vorzugsweise 3 bis 6 Gew.-%, besonders bevorzugt 3 bis 4 Gew.-% partikulärer Füllstoff,
< 2,5 Gew.-%, vorzugsweise < 1,5 Gew.-%, besonders bevorzugt 0,05 bis 1,0 Gew.-% Initiator für die radikalische Polymerisation,
< 2,5 Gew.-%, vorzugsweise < 2 Gew.-%, besonders bevorzugt 0,1 bis 1,2 Gew.-% Stabilisator, und
< 0,3 Gew.-%, vorzugsweise < 0,1 Gew.-%, besonders bevorzugt 0,001 bis 0,08 Gew.-% Pigmente, jeweils bezogen auf die Gesamtmasse des Kernmaterials bzw. des ungehärteten Ausgangsmaterials.

Besonders bevorzugt sind Materialien, die alle Komponenten in den angegebenen bevorzugten Mengen enthalten.

Ein ganz besonders bevorzugtes polymerisierbares Kernmaterial weit folgende Zusammensetzung auf:

| | |
|---|---|
| Bis-GMA | 25,0 - 40,0 Gew.-% |
| Triethylenglykoldimethacrylat | 6,0 - 10,0 Gew.-% |
| Urethandimethacrylat | < 1,0 Gew.-% |
| Decanmethylenglykoldimethacrylat | < 1,0 Gew.-% |
| Hochdisperses Siliciumdioxid | 3,0 - 8,0 Gew.-% |
| Glasfasern, silanisiert | 43,0 - 70,0 Gew.-% |
| Katalysatoren und Stabilisatoren | < 0,5 Gew.-% |
| Pigmente | < 0,1 Gew.-% |

Alle Prozentangaben beziehen sich auf die Gesamtmasse des polymerisierbaren Kernmaterials.

Gemäß einer besonders bevorzugten Ausführungsform ist der faserförmige Füllstoff mit dem polymerisierbaren Matrixmaterial, dem Initiator, ggf. dem partikulären Füllstoff und weiteren Komponenten imprägniert, und kann damit direkt vom Zahnarzt oder Zahntechniker verarbeitet, d.h. geformt und gehärtet werden. Diese vorimprägnierten Materialien weisen vorzugsweise die Form von Stäbchen oder Matten auf.

Als Verblendmaterial werden vorzugsweise pastenförmige polymerisierbare Materialien verwendet, die ein polymerisierbares Bindemittel, Initiator für die radikalische Polymerisation und ggf. Füllstoff, vorzugsweise organischen Füllstoff enthalten. Verblendmaterialien, die frei von anorganischem Füllstoff sind, sind besonders bevorzugt. Die Verblendmaterialien enthalten keinen faserförmigen Füllstoff. Als Bindemittel, Initiatoren und ggf. Füllstoffe sind die oben genannten Materialien bevozugt, die auch zur Herstellung der Kernmaterialien eingesetz werden.

Als organische Füllstoffe eignen sich besonders organische Kunststoffpartikel, insbesondere vorgehärtete Kunststoffpartikel, d.h. teilweise polymerisierte Partikel, die noch über radikalisch polymerisierbare Gruppen verfügen. Gemäß einer besonders bevorzugten Ausführungsform basieren diese Partikel auf den gleichen Monomeren wie das zur Herstellung des Polymermaterials verwendete Bindemittel, so daß Füller und Bindemittel nach der Härtung im Wesentlichen die gleiche Zusammensetzung aufweisen. Im Schliffbild des gehärteten Polymermaterial ist in solchen Fällen der organische Füllstoff zwar sichtbar, hat jedoch nur einen geringen Einfluß auf den E-Modul oder die Steifigkeit des Materials und wirkt damit wie ein nichtverstärkender Füllstoff. Die Materialien haben damit praktisch die gleichen Eigenschaften wie Materialien, die ohne Füllstoff hergestellt wurden. Die Verwendung solcher Füllstoffe dient in erster Linie zur Einstellung der Viskosität und des Polymerisationsschrumpfes des Materials.

Unabhängig voneinander wählbare bevorzugte Mengenbereiche für die einzelnen Komponenten des Verblendmaterials sind:
50 bis 80 Gew.-%, vorzugsweise 50 bis 70 Gew.-%, besonders bevorzugt 60 bis 70 Gew.-% organischen Füllstoff,
20 bis 50 Gew.-%, vorzugsweise 30 bis 50 Gew.-%, besonders bevorzugt 30 bis 40 Gew.-% polymerisierbares Bindemittel,
< 2 Gew.-%, vorzugsweise < 1,5 Gew.-%, besonders bevorzugt 0,1 bis 1,0 Gew.-% Initiator für die radikalische Polymerisation und
0 bis 1 Gew.-%, vorzugsweise 0,05 bis 1,0 Gew.-%, besonders bevorzugt 0,1 bis 0,7 Gew.-% Aktivator für die radikalische Polymerisation, jeweils bezogen auf die Gesamtmasse des Verblendmaterials.

Besonders bevorzugt sind Verblendmaterialien, die alle Komponenten in den angegebenen bevorzugten Mengen enthalten.

Ein ganz besonders bevorzugtes Verblendmaterial auf der Basis anorganischer Füllstoffe weist folgende Zusammensetzung auf:

| | |
|---|---|
| Bis-GMA | < 10,0 Gew.-% |
| Urethandimethacrylat | < 10,0 Gew.-% |
| Decanmethylenglykoldimethacrylat | < 5,0 Gew.-% |
| Hochdisperses Siliciumdioxid | 5,0 - 20,0 Gew.-% |
| Barium-Glasfüller, silanisiert | 35,0 - 60,0 Gew.-% |
| Mischoxid, silanisiert | 15,0 - 25,0 Gew.-% |
| Katalysatoren und Stabilisatoren | < 1,0 Gew.-% |
| Pigmente | < 0,1 Gew.-% |

Alle Prozentangaben beziehen sich auf die Gesamtmasse des Verblendmaterials.

Füllstoffhaltige Verblendmaterialien werden vorzugsweise in Form von zwei Komponenten bereitgestellt, einer festen Komponente und einer flüssigen Komponente, wobei die feste Komponente den organischen Füllstoff und vorzugsweise den Initiator für die radikalische Polymerisation und die flüssige Komponente das polymerisierbare Bindemittel und gegebenenfalls den Aktivator für die radikalische Polymerisation enthält.

Ein besonders bevorzugtes zweikomponentiges Verblendmaterial auf der Basis von organischem Füllstoff weist folgende Zusammensetzung auf:

| Pulverkomponente: | |
|---|---|
| PMMA | > 98,0 Gew.-% |
| Benzoylperoxid, Pigmente | < 2,0 Gew.-% |

| Flüssige Komponente: | |
|---|---|
| Methylmethacrylat | 60,0 - 90,0 Gew.-% |
| Ethylenglycoldimethacrylat | 5,0 - 40,0 Gew.-% |
| Amin (Diethanol-p-Toluidin) | 0 - 1,0 Gew.-% |

Die Prozentangaben beziehen sich jeweils auf die Gesamtmasse der pulverförmigen bzw. flüssigen Komponente.

Das härtbare Matrixmaterial und das härtbare Verblendmaterial können durch Polymerisation, vorzugsweise durch radikalische Polymerisation gehärtet werden.

Gegenstand der Erfindung sind auch Kits zur Herstellung von Dentalprothesen. Die erfindungsgemäßen Kits enthalten mindestens ein härtbares Kernmaterial, vorzugsweise 2 bis 5 unterschiedliche Kernmaterialien, z.B. unterschiedlich geformte Kernmaterialien, und mindestens ein härtbares Verblendmaterial, vorzugsweise 1 bis 5 unterschiedlich Verblendmaterialien, z.B. unterschiedlich gefärbte Kernmaterialien. Darüber hinaus enthält der Kit vorzugsweise auch künstliche Zähne, die auf der Prothesenbasis aufgestellt werden können. Kits, die Kern- und Verblendmaterialien mit der oben angegebenen Zusammensetzung enthalten, sind besonders bevorzugt.

Die härtbaren Kernmaterialien werden vorzugsweise in Form von unterschiedlich dimensionierten Stäbchen und Matten eingesetzt, in denen der faserförmige Füllstoff mit Bindemittel, Initiator und ggf. dem partikulärem Füllstoff imprägniert ist, und die nach Bedarf der Verpackung entnommen, geschnitten und geformt werden können. Einkomponentige Kernmaterialien, das sind Materialien, die direkt, d.h. ohne Zugabe einer zweiten Komponente, gehärtet werden können, beispielsweise durch Wärme oder Licht, sind besonders bevorzugt.

Die Vermessung und Konstruktion der Klammern erfolgt auf an sich bekannte Weise (vgl. z.B. Reinhard Marxkors, Lehrbuch der zahnärztlichen Prothetik, 3. Auflage, Deutscher Zahnärzte Verlag) und richtet sich nach den individuellen Gegebenheiten der jeweiligen Mundsituation. Im allgemeinen weisen die erfindungsgemäßen Verankerungselemente eine runde bis halbkreisförmige Querschnittsform auf, wobei unter "halbkreisförmig" auch solche Querschnittsformen verstanden werden, die mehr oder weniger als eine halbe Kreisfläche umfassen. Die Querschnittsformen der Klammern können sich auch von ellipsenförmigen Flächen ableiten, also beispielsweise die Form einer halben Ellipse ausweisen.

Gegenstand der Erfindung ist auch ein Verfahren zur Herstellung von Dentalprothesen mit metallfreien Verankerungselementen bei dem eine Prothesenbasis mit Verankerungselementen aus einem wie oben definierten Kernmaterial versehen und die Verankerungselemente dann ggf. mit einem Verblendmaterial verblendet und gehärtet werden. Die Verankerungselemente können gleichzeitig mit der Prothesenbasis oder im Anschluß daran geformt werden. Kernmaterial und Verblendmaterial können nach der Formung der Verankerungselemente zusammen, beispielsweise durch Erwärmen gehärtet werden. Alternativ kann die Härtung nacheinander erfolgen, indem zunächst das Kernmaterial gehärtet und dann das Verblendmaterial aufgebracht und gehärtet wird. Bei der Verwendung von mehr als einem Kernmaterial oder mehr als einem Verblendmaterial kann die Härtung der einzelnen Materialien schichtweise erfolgen. Anschießend wird die Prothese in üblicher Weise durch Anbringen der Ersatzzähne fertiggestellt.

Aus ästhetischen Gründen weisen die Klammerquerschnitte in der Zahnhorizontalen vorzugsweise eine Dimension von maximal 1 mm auf. Zur Gewährleistung der gewünschten mechanischen Eigenschaften beträgt die minimale Dimension in horizontaler Richtung vorzugsweise 0,6 mm.

In vertikaler Richtung betragen die Abmessungen des Klammerquerschnitts vorzugsweise 1 bis 2 mm, wobei die Klammern am Ende spitz zulaufen können und im Bereich der Klammerbasis, d.h. im Bereich des Zusammentreffens mit der Prothese, stärker ausfallen können.

Bei verblendeten Verankerungselementen beträgt die Dicke der Schicht des Verblendmaterials vorzugsweise mindestens 0,1 mm, besonders bevorzugt 0,1 bis 0,2 mm.

Die beschriebenen Werkstoffe, d.h. Kern- und Verblendmaterialien, eignen sich besonders zur Herstellung von Verankerungselementen von dentalen Prothesen. Darüber hinaus können sie vorteilhaft auch zur Herstellung von Verbindungselementen, wie z.B. Sublingualbügeln, aber auch zur Herstellung der Prothesensättel eingesetzt werden.

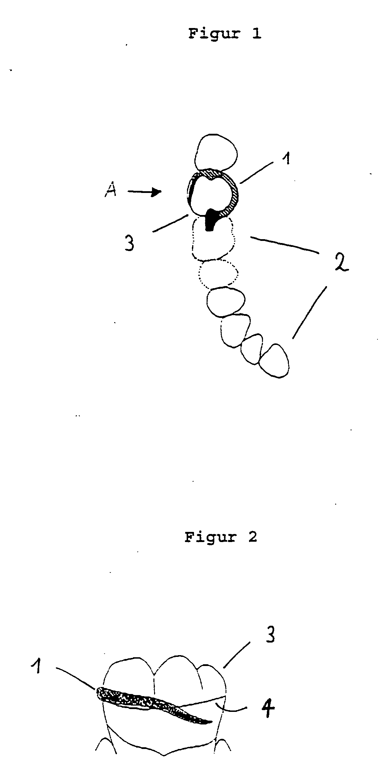

**Figur 1** zeigt die Verankerung einer Prothese an einem natürlichen Zahn in Draufsicht. Die Klammer **1** der Prothese **2** umgreift den Verankerungszahn **3** in einer horizontalen Ebene. Um einen sicheren Halt der Prothese zu gewährleisten, muß die Klammer fest am Zahn anliegen und darf keine Bewegung der Prothese erlauben.

**Figur 2** zeigt die Klammer in der Seitenansicht (Blickrichtung A). Die Klammer **1** ist hier so angeordnet, daß sie den Zahn **3** unterhalb des Zahnäquators **4**, d.h. des größten Querschnitts des Zahns, umfaßt. Auf diese Weise wird die Prothese gegen vertikal wirkende Zugkräfte gesichtert.

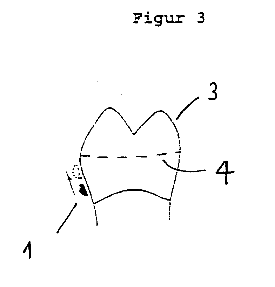

Zur Entfernung der Prothese muß die Klammer aufgebogen werden, damit sie über den Äquator **4** des Zahns **3** geführt werden kann (**Figur 3**). Bereits kleine Aufbiegungen können plastische Verformungen und Ermüdungen von Metallklammern bewirken. Die erfindungsgemäßen Kunststoffklammern zeichnen sich demgegenüber durch ein hohes Maß an Elastizität aus, und können so deutlich höhere Aufbiegungen ohne plastische Verformung überstehen als Metallklammern. Mit den erfindungsgmäßen Klammern lassen sich problemlos Aufbiegungen von mehr als 1,5 mm erzielen, was eine deutliche Verbesserung gegenüber Metallklammern darstellt, die üblicherweise Aufbiegungen von 0,2 bis 0,6 mm zulassen.

Die Aufbiegung wird an halbkreisförmigigen Standardklammern mit einem Radius von 4 mm gemessen, die aus Metall- bzw. Kunststoffdraht mit rundem Querschnitt und einem Durchmesser von 1 mm geformt sind. Es wird die maximale Aufbiegung ermittelt, d.h. die ohne irreversible Verformung oder mechanische Beschädigung der Klammer maximal mögliche Aufbiegung.

Die Aufbiegung ist ein Maß für die geometrische Verformbarkeit der Klammern beim Einsetzen bzw. Herausnehmen. Je größer die Differenz der Aufbiegung zum Unterschnitt ist, desto größer ist die Gebrauchssicherheit. Dies ist besonders auch dann von Vorteil, wenn die Prothese beim Einsetzen oder auf sonstige Weise verkanntet wird, da das Risiko einer Beschädigungen der Klammer deutlich reduziert wird.

Als Unterschnitt bezeichnet man in der Prothetik den geometrischen Unterschied (Differenz) zwischen dem Äquator eines Zahns (Maximaler Durchmesser) und dem Auflagebereich der Klammer.

Andererseits weisen die erfindungsgemäßen Klammern eine deutlich höhere Festigkeit auf, als die bekannten Acetalklammern und gewährleisten so einen sehr viel sicheren Sitz der Prothese als diese, so daß sich vorteilhaft die positiven Eigenschaften von Metall- und Acetalklammern gemeinsam verwirklichen lassen.

Die Versorgung eines Lückengebisses durch eine Teilprothese beginnt mit der Herstellung der Basis. Nach der Vorbereitung des Restgebisses (Füllungen, Parodontalbehandlung) werden Situationsmodelle erstellt, auf denen der Zahnarzt nach dem Vermessen einen Entwurf der Basis skizziert. Häufig zeigt sich dabei die Notwendigkeit, bei den dafür vorgesehenen Zähnen durch Präparation kleiner Kavitäten Platz für die Aufnahme okklusaler Aufruhen zu schaffen, um eine Störung des Zahnreihenschlusses durch die Abstützung zu vermeiden.

Nach erneuter Situationsabformung und Modellherstellung erfolgt das endgültige Einzeichnen des Entwurfs der Basis und ihre Herstellung im Labor. Die Basis wird am Patienten auf einwandfreien Sitz überprüft. Für das nun folgende Aufstellen der künstlichen Zähne müssen die Modelle durch eine Bißnahme in den Artikulator montiert werden, wobei man vorteilhafterweise die Basis als Bißschablone benutzt, nachdem man sie im Bereich des oder der Sättel mit einem Wachswall versehen hat. Nach der Anprobe der Basis mit den in Wachs aufgestellten Zähnen wird die Prothese fertiggestellt und eingegliedert. Mehrere Prothesensättel werden durch Kunststoffbügel miteinander verbunden.

Die Prothesenbasis wird vorzugsweise ebenfalls ohne die Verwendung von Metallen hergestellt. Ein bevorzugtes Material hierfür ist Polymethyl(meth)acrylat (PMMA), in das die Ersatzzähne und die Verankerungselemente eingesetzt werden. Die Ersatzzähne basieren vorzugsweise ebenfalls auf PMMA. Gemäß einer weiteren, bevorzugten Ausführungsform erfolgt die Herstellung der Prothesenbasis, d.h. der Sättel und Verbindungselemente unter Verwendung der oben beschriebenen Kern- und Verblendmaterialien, wobei die Verblendmaterialen vorzugsweise zahnfleischfarben eingefärbt sind.

Die erfindungsgemäßen Prothesen bzw. Prothesenteile weisen die für die Kaubelastung erforderliche Festigkeit aber auch die nötige Flexibilität für das tägliche Wechseln der Prothese auf. Es wurde gefunden, daß Befestigungselemente aus faserverstärktem Kompositmaterial eine sichere Befestigung von Teilprothesen ermöglichen ohne die Nachteile von Metall- oder Acetalklammern zu zeigen. Die erfindungsgemäßen Dentalprothesen weisen damit eine Kombination von Eigenschaften auf, die für den gewünschten Einsatzzweck eine hohe Anwendungssicherheit gewährleistet und eine unerwartete Verbesserung gegenüber dem Stand der Technik darstellt.

Im folgenden wird die Erfindung anhand von Ausführungsbeispielen beschrieben.

### Beispiele

### Beispiel 1: Bestimmung der mechanischen Eigenschaften von Prüfkörpern

Zur Bestimmung der mechanischen Eigenschaften der untersuchten Materialien wurden stabförmige Prüfkörper mit einer Breite von 4 mm hergestellt. Wenn nicht anders angegeben betrug die Gesamtdicke 1,05 mm. Prüfkörper aus verblendeten, faserverstärkten Kunststoffen bestanden aus Kernmaterial, das auf Ober- und Unterseite mit Polymermaterial beschichtet wurde. Die Dicke der Schicht des fasergefüllten Kernmaterials betrug 0,75 mm, die Dicke der Verblendschichten jeweils 0,15 mm.

Es wurden der Biege-E-Modul und die maximale Dehnung gemäß DIN/ISO 178 gemessen. Die Ergebnisse sind in Tabelle 1 gezeigt. Die ebenfalls aufgeführte Aufbiegung wurde an halbkreisförmigen Standardklammern mit einem Klammerradius von 4 mm und einem Materialdurchmesser der Klammerarme von 1 mm gemessen. In der Tabelle ist die maximale Aufbiegung angegeben, die ohne irreversible Verformung und mechanische Beschädigung der Klammer erzielt werden konnte.

Zur Herstellung der Prüfkörper wurden die folgenden Materialien verwendet:

| **Polymerisierbares Kernmaterial** | |
|---|---|
| **Bestandteil** | **Anteil (Gew.-%)** |
| Bis-GMA | 24,5 |
| Triethylenglykoldimethacrylat (TEGDMA) | 6,2 |
| Urethandimethacrylat (UDMA) | < 1,0 |
| Decanmethylenglykoldimethacrylat | < 1,0 |
| Hochdisperses Siliciumdioxid | 3,5 |
| Glasfasern, silanisiert | 65,0 |
| Katalysatoren und Stabilisatoren | < 0,6 |

| **Verblendmaterial (mit organischem Füllstoff)** | |
|---|---|
| **Bestandteil** | **Anteil (Gew.-%)** |
| **Pulverkomponente** | |
| PMMA | > 98,0 |
| Benzoylperoxid, Pigmente | < 2,0 |

| **Flüssige Komponente** | |
|---|---|
| Methylmethacrylat | 66,5 |
| Ethylenglycoldimethacrylat | 33,0 |
| Diethanol-p-Toluidin | 0,5 |

Pulverkomponente und Flüssigkomponente wurden im Verhältnis von 2,5 : 1 miteinander gemischt. Nach der Polymerisation waren die PMMA-Partikel der Pulverkomponente im Schliffbild des gehärteten Materials wie ein Füllstoff sichtbar, hatten jedoch keinen Einfluß auf den E-Modul oder die Steifigkeit. Die Pulverkomponente wirkt wie ein nicht verstärkender Füllstoff.

| **Verblendmaterial mit anorganischem Füllstoff** | |
|---|---|
| **Bestandteile Bestandteile** | **Anteil (Gew.-%)** |
| Bis-GMA | 9,0 |
| Urethandimethacrylat | 9,3 |
| Decanmethylenglykoldimethacrylat | 4,8 |
| Hochdisperses Siliciumdioxid | 11,8 |
| Barium-Glasfüller, silanisiert | 46,2 |
| Mischoxid, silanisiert | 12,2 |
| Katalysatoren und Stabilisatoren | < 1,0 |
| Pigmente | 0,1 |

**Tabelle 1**

| **Messung der Materialwerte (Prüfstäbe)** | | | | |
|---|---|---|---|---|
| **Material** | **Biege-E-Modul [GPa]** | **Max. Dehnung [%]** | **Flexibilität**^{**1)**} **[10**^{**-3**} **·GPa**^{**-1**}**]** | **Aufbiegung [mm]** |
| Kernmaterial | 36 | 2,3 | 0,64 | 2,0 |
| Verblendmaterial (anorg. Füllstoff) | 10 | 1,8 | 1,80 | n.m. |
| Verblendmaterial (org. Füllstoff) | 2 | 3,5 | 17,50 | n.m. |
| Co/Cr-Legierung²⁾ | 218 | 0,25 | 0,01 | 0,2 |
| Au-cast³⁾ | 98 | 0,36 | 0,04 | 0,3 |
| Au-hardened⁴⁾ | 103 | 0,57 | 0,06 | 0,6 |
| Acetal-Harz | 3 | 2,3 | 7,67 | 1,8 |
| Kernmaterial (0,75 mm) beidseitig verblendet (2 x 0,15mm) (anorg. Füllstoff) | 16 | 2,3 | 1,44 | 1,7 |
| Kernmaterial (0,75 mm) beidseitig verblendet (2 x 0,15mm) (org. Füllstoff) | 13 | 3,1 | 2,38 | 2,4 |

| | | | | |
|---|---|---|---|---|
| ¹⁾ maximale Dehnung in %/Biege-E-Modul in GPa | | | | |
| ^{2) - 4)} es wurden Materialien von üblicher Dentalqualität eingesetzt n.m. nicht gemessen | | | | |

## Patentansprüche

1. Dentalprothese, **dadurch gekennzeichnet, daß** sie mindestens ein metallfreies Verankerungselement aus Kunststoffmaterial mit einem Biege-E-Modul von mindestens 10 GPa und einer maximalen Dehnung von mindestens 0,8 % aufweist.

2. Dentalprothese nach Anspruch 1, **dadurch gekennzeichnet, daß** das Kunststoffmaterial einen Biege-E-Modul von 10 bis 80 GPa und eine maximale Dehnung von 0,8 bis 4 % aufweist.

3. Dentalprothese nach Anspruch 1 oder 2, **dadurch gekennzeichnet, daß** Biege-E-Modul und maximale Dehnung in einem solchen Verhältnis zueinander stehen, daß der Quotient aus maximaler Dehnung in % und Biege-E-Modul in GPa im Bereich von 0,4 · 10⁻³ bis 15 · 10⁻³ GPa⁻¹ liegt.

4. Dentalprothese nach einem der Ansprüche 1 bis 3, **dadurch gekennzeichnet, daß** das Kunststoffmaterial ein polymeres Matrixmaterial enthält, in das ein faserförmiger Füllstoff eingebettet ist (Kernmaterial).

5. Dentalprothese nach Anspruch 4, **dadurch gekennzeichnet, daß** das Kernmaterial ein Biege-E-Modul von mehr als 20 GPa und eine maximalen Dehnung von mehr als 1 % aufweist.

6. Dentalprothese nach einem der Ansprüche 1 bis 5, **dadurch gekennzeichnet, daß** das Kernmaterial 43 bis 70 Gew.-% faserförmigen Füllstoff enthält.

7. Dentalprothese nach einem der Ansprüche 1 bis 6, **dadurch gekennzeichnet, daß** das faserförmigen Füllstoff enthaltende polymere Matrixmaterial mit einem Polymermaterial ohne faserförmigen Füllstoff verblendet ist (Verblendmaterial).

8. Dentalprothese nach Anspruch 7, **dadurch gekennzeichnet, daß** das Verblendmaterial ein Biege-E-Modul von 2 bis 15 GPa und eine maximale Dehnung von mehr als 1% aufweist.

9. Dentalprothese nach Anspruch 7 oder 8, **dadurch gekennzeichnet, daß** das Verblendmaterial keinen anorganischen Füllstoff enthält.

10. Dentalprothese nach einem der Ansprüche 1 bis 9, **dadurch gekennzeichnet, daß** das Verblendmaterial organischen Füllstoff enthält.

11. Kit zur Herstellung einer Dentalprothese gemäß den Ansprüchen 1 bis 10, **dadurch gekennzeichnet, daß** er ein härtbares Kernmaterial und ein härtbares Verblendmaterial enthält.

12. Kit nach Anspruch 11, **dadurch gekennzeichnet, daß** das härtbare Kernmaterial
| | |
|---|---|
| 25 bis 52 Gew.-% | polymerisierbares Bindemittel, |
| 43 bis 70 Gew.-% | faserförmigen Füllstoff, |
| 3 bis 8 Gew.-% | partikulären Füllstoff, |
| < 2,5 Gew.-% | Initiator für die radikalische Polymerisation, |
| < 2,5 Gew.-% | Stabilisator, und |
| < 0,3 Gew.-% | eines oder mehrerer Pigmente |
enthält.

13. Kit nach einem der Ansprüche 11 bis 12, **dadurch gekennzeichnet, daß** das Verblendmaterial
| | |
|---|---|
| 50 bis 80 Gew.-% | organischen Füllstoff, |
| 20 bis 50 Gew.-% | polymerisierbares Bindemittel, |
| < 2 Gew.-% | Initiator für die radikalische Polymerisation und |
| 0 - 1 Gew.-% | Beschleuniger |
enthält.

14. Kit nach Anspruch 10, **dadurch gekennzeichnet, daß** das Verblendmaterial mindestens eine feste Komponente und mindestens eine flüssige Komponente umfaßt, wobei die feste Komponente den organischen Füllstoff und den Initiator und die flüssige Komponente das Bindemittels und gegebenenfalls den Aktivator enthält.

15. Kit nach einem der Ansprüche 11 bis 14, dadurch gegenzeichnet, daß er 1 bis 5 unterschiedliche Kernmaterialien und/oder 1 bis 5 unterschiedliche Verblendmaterialien enthält.

16. Verfahren zur Herstellung von Dentalprothesen mit metallfreien Verankerungselementen, **dadurch gekennzeichnet, daß** eine Prothesenbasis mit Verankerungselementen aus einem Kernmaterial versehen wird, die Verankerungselemente anschließend ggf. mit einem Verblendmaterial verblendet und Kern- und Verblendmaterial gleichzeitig oder nacheinander gehärtet werden.
